# EUROPÄISCHE PATENTANMELDUNG

(11) **EP 1 762 607 A1**
(43) Veröffentlichungstag der Anmeldung: **14.03.2007**
(21) Anmeldenummer: 05019404.2
(22) Anmeldetag: 07.09.2005
(51) Int. Cl.: C12M 1/107, C12M 1/36, C12P 5/02

(54) **Biogasanlagen-Regelungsverfahren**

(71) Anmelder: U.T.S. Umwelt-Technik-Süd GmbH, 84419 Obertaufkirchen (DE)
(72) Erfinder: Bürger, Adam, 84405 Grüntegernbach (DE); Niederbacher, Michael, 39031 Bruneck (BZ) (IT)
(74) Vertreter: Neubauer, Hans-Jürgen

(57) **Zusammenfassung**

Die Erfindung betrifft ein Biogasanlagen-Regelungsverfahren für eine Biogasanlage, die aus einem Fermenterbehälter wenigstens einer Basis-Zudosier-Vorrichtung (8) für eine Basis-Biomasse (9) und einer Regeleinrichtung zur Regelung wenigstens eines Prozessparameters als Regelgröße des Fermentationsprozesses besteht. Erfindungsgemäß wird vorgeschlagen, dass die Ausregelung einer Regelabweichung bezüglich wenigstens eines Prozessparameters durch eine Regel-Zudosierung einer bestimmten Menge einer zugeordneten und den jeweiligen Prozessparameter beim Fermentationsprozess beeinflussenden Regel-Biomasse (20a, 20b, 20c, 20d) erfolgt, die für einen Regeleingriff in einem Regel-Vorratsbehälter vorgehalten wird und mittels einer Regel-Zudosier-Vorrichtung (21a, 21b, 21c, 21d) als Stellglied entsprechend einem Stellsignal zudosiert wird.

## Beschreibung

Die Erfindung betrifft ein Biogasanlagen-Regelungsverfahren nach dem Oberbegriff des Anspruchs 1.

Bedingt durch staatlich festgelegte Vergütungssätze für elektrischen Strom aus regenerativen Energien, gewinnt die Biogasgewinnung zur Energieerzeugung vorrangig im landwirtschaftlichen Bereich zunehmend an Bedeutung.

Die dafür bekannten Biogasanlagen sind meist für einen kontinuierlichen, einstufigen Betrieb ausgelegt und bestehen aus einem Fermenterbehälter, in dem in einem anaeroben Nassvergärungsprozess zur Gewinnung von Biogas Biomassen als organische Substrate vergoren werden. Je nach den Gegebenheiten können einem solchen Fermenterbehälter ein Nachfermenter und ein Endlager sowie separate Gasspeicher für das erzeugte Biogas nachgeordnet sein. Zur Beschickung des Fermenterbehälters ist eine Basis-Zudosier-Vorrichtung vorgesehen, wobei für flüssige Substratbestandteile Pumpen und für feste Biomassen Feststoff-Zudosiereinrichtungen mit Wägeeinrichtungen und Befüllschnecken oder Förderbändern verwendet sind. Zudem sind in den Behältern und Fermentern Rühreinrichtungen angebracht (EP 1 251 165 A1).

Die Erzeugung von Biogas erfolgt bei der anaeroben Nassvergärung im wesentlichen in vier Schritten:

Im ersten Schritt, der Hydrolyse, werden komplexe Verbindungen im organischen Substrat in einfachere organische Verbindungen auf biochemischem Wege durch von Bakterien freigesetzten Enzymen zerlegt.

Im zweiten Schritt, der Versäuerungsphase (Acidogenese), werden die gebildeten Zwischenprodukte durch säurebildende Bakterien weiter zu niederen Festsäuren sowie Kohlendioxid und Wasserstoff abgebaut.

Im dritten Schritt, der Essigsäurebildung (Acetogenese), werden diese Produkte durch Bakterien zu Vorläufersubstanzen des Biogases umgesetzt. Für diese Bakterien ist ein hoher Wasserstoffgehalt schädlich, so dass sie mit den nachfolgenden Methanbakterien eine enge Lebensgemeinschaft eingehen müssen, da diese bei der Bildung von Methan Wasserstoff verbrauchen.

Im vierten und letzten Schritt, der Methanogenese, wird aus den Produkten der Acetogenese von Methanbakerien Methan gebildet.

Dadurch ergibt sich im Fermenterbehälter oberhalb des Substratspiegels Biogas mit einer Zusammensetzung von etwa 50 bis 75 Vol.-% Methan, 25 bis 45 Vol.-% Kohlendioxid, 2 bis 7 Vol.-% Wasser sowie weiterer Anteile in geringer Konzentration, insbesondere Schwefelwasserstoff, Stickstoff, Sauerstoff und Wasserstoff.

Ein wesentlicher Aspekt der Biogasgewinnung ist die Nutzung zur Einspeisung in ein Blockheizkraftwerk für eine wirtschaftlich lukrative Energieerzeugung. Dort wird in einem Gasmotor Biogas verbrannt, dessen Energiegehalt in mechanische Drehenergie und anschließend durch einen Generator in elektrische Energie zur weiteren Verwertung umgewandelt wird.

Bei einem bevorzugt einstufigen Verfahren laufen die vorstehend genannten vier Abbauschritte gemeinsam in einem Fermenterbehälter ab. Für eine angestrebte hohe Biogaserzeugungsrate und die davon abhängige hohe Erzeugungsrate von elektrischer Energie ist es erforderlich, die Milieubedingungen für die Bakterien möglichst optimal zu gestalten.

Dazu wird die Temperatur im Fermenterbehälter je nach Verfahrensführung auf einen mesophilen Temperaturbereich, etwa zwischen 32 und 42°C oder einen thermophilen Temperaturbereich, etwa zwischen 50 und 57°C geregelt. Die Nährstoffversorgung für die Bakterien wird durch eine bestimmte vorgegebene, meist chargenförmige Beschickung mittels der Basis-Zudosiervorrichtung mit teilweise flüssigem Substrat und Biomassefeststoffen vorgenommen. Weiter ist es erforderlich, den pH-Wert zu überwachen und gegebenenfalls zu korrigieren. Zudem ist es erforderlich, den Fermenterinhalt geeignet zu durchmischen, um einen intensiven Kontakt von Bakterien und Substrat zu ermöglichen. Weiter ist die Biogaserzeugungsrate auch abhängig von der hydraulischen Verweilzeit des Substrats im Fermenter. Eine solche Biogasanlage und ein Verfahren zu deren kontinuierlichem, einstufigem Betrieb zur Erzeugung von Biogas durch anaerobe Nassvergärung ist beispielsweise aus der Schrift "Handreichung, Biogasgewinnung und -Nutzung, Bundesministerium für Verbraucherschutz, Ernährung und Landwirtschaft, Fachagentur nachwachsende Rohstoff e. V." bekannt. Hierin werden auch die Prozessparameter erläutert, die zu einem stabilen Prozessablauf und einer hohen Biogasausbeute führen oder sich störend auswirken:

### Temperatur

Da die Bakterien beim Abbau von Biomasse nur geringe Mengen an Eigenwärme produzieren, die nicht für das Erreichen der prozessoptimalen Umgebungstemperatur ausreicht, muss beim üblichen mesophilen oder thermophilen Prozessbetrieb der Fermenterbehälter isoliert und von extern beheizt werden. Dazu ist eine auf einen vorgegebenen Temperatur-Sollwert geregelte Heizung bekannt und üblich.

### pH-Wert

Die Essigsäure und Methan bildenden Bakterien benötigen einen pH-Wert im neutralen Bereich bei ca. 6,8 bis 7,5. Ein solcher pH-Wert stellt sich regelmäßig durch die alkalischen und sauren Stoffwechselprodukte während des anaeroben Abbaus selbsttätig ein. Es handelt sich hierbei jedoch um ein empfindliches pH-Gleichgewicht, welches zwar durch das freigesetzte Kohlendioxid im neutralen Bereich gepuffert ist, jedoch mit nur geringer Pufferkapazität. Beim Absinken des pH-Werts kommt es zusätzlich zu einer Anhäufung der Säuren aus der Acidogenese, was ein Absinken des pH-Werts weiter beschleunigt. Dies kann im schlimmsten Fall einen kompletten Austausch des Fermenterinhalts mit erheblichen wirtschaftlichen Verlusten erforderlich machen. Um dies zu verhindern ist es bekannt, eine pH-Wert-Erhöhung in den neutralen Bereich durch Zugabe von Laugen, insbesondere von Kalilaugen vorzunehmen. Die Zugabe solcher Chemikalien ohne ein damit verbundenes Energieerzeugungspotential stellt einen unerwünschten Eingriff in den biologischen Abbauprozess von Biomassen dar.

### Hemmstoffe

Weiter ist es aus der vorstehend genannten Schrift bekannt, dass sich Inhaltsstoffe von Substraten in zu hohen Konzentrationen schädlich auf die Bakterien und damit auf die Biogaserzeugung auswirken können. Solche Inhaltsstoffe können somit als Hemmstoffe in bestimmten Konzentrationen den Gärprozess schädigen, wobei auch Wechselwirkungen unter solchen Hemmstoffen auftreten können. Insbesondere wirkt sich bekanntermaßen Ammoniak (NH₃) schon in geringen Konzentrationen schädigend auf die Bakterien aus. Ammoniak steht dabei im Gleichgewicht mit der Ammoniumkonzentration (NH₄), wobei dieses Gleichgewicht auch in Beziehung zum vorherrschenden pH-Wert und zur Temperatur liegt. Auch Schwefelwasserstoff (H₂S) als weiteres Produkt des Gärprozesses kann schon in geringen Konzentrationen den Abbauprozess hemmen. Als weitere Hemmstoffe sind beispielsweise konzentrationsabhängig Natrium, Kalium, Kalzium, Magnesium sowie Schwermetalle und verzweigte Fettsäuren bekannt.

In der vorstehenden Schrift ist auch der derzeitige Stand der Automatisierung von Biogasanlagen zusammengefasst, wobei Steuerungen und/oder Regelungen, zum Teil visualisierte, computergestützte Regelungen mit Fernüberwachung für die Komponenten Substratbeschickung, gegebenenfalls Hygenisierung, Fermenterheizung, Mischaggregate, Sedimentaustrag, Substrattransport durch die Anlage, Entschwefelung und Blockheizkraftwerk bekannt sind.

Weiter wird auf Entwicklungen für kontinuierliche Messsysteme hingewiesen, mit denen vorzugsweise die Prozessparameter Prozesstemperatur, Art der zugeführten Substrate, pH-Wert, Gasmenge und Gaszusammensetzung sowie kurzkettige Fettsäuren zumindest täglich überwacht werden sollen. Eine Langzeitüberwachung in Verbindung mit einer Dokumentation soll nach einer gewissen Überwachungszeit Normalwerte für Prozessparameter bei einem stabil laufenden Prozess erbringen mit der Möglichkeit eines Eingriffs bei erkennbaren Abweichungen. Als mögliche Eingriffe außer einer Temperaturregelung in den Prozessablauf sind explizit nur die bereits vorher genannte Zugabe von Laugen bei der Gefahr einer Übersäuerung des Prozesses genannt oder für den gleichen Zweck eine Reduzierung der Substratzugabe um den Methanbakterien Zeit zu geben, vorhandene Säuren abzubauen. Ansonsten wird bei der Prozessführung der Gärprozess weitgehend sich selbst überlassen unter der bekannten Annahme, dass sich die Bakterien substratspezifisch entwickeln, d. h. nach einer gewissen Anfahrzeit Bakterienstämme die Oberhand gewinnen, die für den Abbau einer etwa gleichbleibenden Substratzusammensetzung gut geeignet sind und zu einem stabilen Prozess führen. Weitere prozessführende und prozessoptimierende Eingriffe über die vorstehend genannten Maßnahmen hinaus, werden nicht durchgeführt.

Aufgabe der Erfindung ist es, ein Biogasanlagen-Regelungsverfahren vorzuschlagen, mit dem eine stabilere Prozessführung mit höherer Biogasausbeute möglich ist.

Diese Aufgabe wird mit den Merkmalen des Anspruchs 1 gelöst.

Gemäß Anspruch 1 erfolgt die Ausregelung einer Regelabweichung bezüglich wenigstens eines Prozessparameters durch eine Regel-Zudosierung einer bestimmten Menge einer zugeordneten und den jeweiligen Prozessparameter beim Fermentationsprozess beeinflussenden Regel-Biomasse, die für einen Regeleingriff in einem Regelvorratsbehälter vorgehalten wird und mittels einer Regel-Zudosiervorrichtung als Stellglied entsprechend einem Stellsignal zudosiert wird.

Damit wird für eine automatisierte Optimierung einer Prozessführung die Kenntnis verwertet, dass unterschiedliche Substrate als Regel-Biomasse Prozessparameter beim Gärprozess beeinflussen können.

Wenn beispielsweise bei einem eingeschwungenen stabilen Gärprozess mit über längerer Zeit gleicher Substratzugabe ein Ammoniakwert bestimmter, stabiler Größe messtechnisch erfasst wird, kann dieser als Sollwert an der Regelungseinrichtung vorgegeben werden. Bei einem gemessenen Anstieg des Ammoniakwerts gegenüber diesem Sollwert, beispielsweise bedingt durch nicht vermeidbare Variationen im Basis-Substratmix ergibt sich eine gesteigerte Hemmwirkung des Ammoniaks mit einer Störung des Prozesses und damit verbundener reduzierter Biogasausbeute. Um dies zu vermeiden kann durch geregelte Zudosierung eines Substrats mit hohem Eiweißgehalt als Regel-Biomasse das Gleichgewicht zwischen Ammoniak (NH₃) und Ammonium (NH₄) zugunsten des Ammoniums verschoben werden. Dadurch wird die Ammoniakbildung reduziert, so dass die daraus resultierende Hemmwirkung ebenfalls reduziert wird.

Ein weiteres Beispiel für die Erfindung besteht darin, dass eine hohe Biogasausbeute insbesondere dann möglich ist, wenn die als Basis-Biomasse verwendeten Substrate hohe Anteile an Kohlenhydraten, Fetten und Proteinen aufweisen. Es liegt somit nahe, möglichst Substrate zu verwenden, die besonders hohe Konzentrationen dieser Stoffgruppen enthalten. Dies funktioniert aber regelmäßig nur über einen gewissen Zeitraum. Da die Bakterien für ihr Überleben neben diesen Stoffgruppen auch andere Nährstoffe und Spurenelemente benötigen, kann es durch eine entsprechende Unterversorgung der Bakterien zu einer völligen Verarmung des Prozesses, mit einer stark reduzierten Gasausbeute kommen. Auch für diesen Fall kann durch eine regelungstechnisch automatisierte Zudosierung einer Regel-Biomasse, die diese benötigten anderen Nährstoffe und Spurenelemente aufweist, eine solche Prozessverarmung vermieden und eine stabile Prozessführung mit hoher Biogasausbeute erreicht werden. Die Zusammensetzung der Regel-Biomasse muss dabei der die Grundversorgung stellenden Basis-Biomasse als Ergänzungsversorgung angepasst werden.

Ein weiteres Beispiel für das erfindungsgemäße Biogasanlagen-Regelungsverfahren besteht darin, dass bei einer Umstellung des Substratmixes der Basis-Biomasse, beispielsweise durch eine saisonbedingte Umstellung auf andere nachwachsende Rohstoffe, der stabile, eingeschwungene Prozessverlauf erheblich gestört sein kann mit reduzierter Biogasausbeute und im Extremfall mit einem Absterben der Bakterien. Auch für diesen Fall kann bei messtechnisch erfassten Regelabweichungen bei Prozessparametern schnell und wirksam durch Zudosierung von ausgleichenden Regel-Biomassen eine Instabilität des Gärprozesses vermieden oder zumindest weitgehend reduziert werden.

Die vorstehende Optimierung der Prozessführung kann sich durch entsprechende Sollwertvorgaben auch auf die Einhaltung der geringsten Kosten pro produzierter Kilowattstunde beziehen, die eine Biomassemischung haben sollte oder auf die 100%ige Auslastung der Anlage, insbesondere der Motoren, welche über die Regel-Zudosierung geregelt werden kann.

Gemäß Anspruch 2 wird vorgeschlagen, wenigstens zwei unterschiedliche Regel-Biomassen in zugeordneten Regel-Vorratsbehältern vorzuhalten und bedarfsweise geregelt dem Prozess zuzudosieren. Damit ist insbesondere bei einer größeren Anzahl von vorgehaltenen Regel-Biomassen unterschiedlicher Zusammensetzung eine fein abgestimmte gezielte Beeinflussung des Prozesses möglich.

Nach Anspruch 3 ist es zweckmäßig mehrere Prozessparameter gleichzeitig messtechnisch zu erfassen und zu regeln. Da eine Regel-Biomasse bestimmter Zusammensetzung auch mehrere Prozessparameter beeinflussen kann, ist es zweckmäßig, dies bei der Regelung zu berücksichtigen.

Diese gegenseitigen Beeinflussungen werden vorteilhaft gemäß Anspruch 4 in einem Rechner berücksichtigt, wobei diese Beeinflussungen in einem Programm und/oder in einem Kennfeld berücksichtigt werden können. Dabei kann ein solcher Rechner selbst die Funktion eines Reglers aufweisen und dabei einen geeigneten Regelalgorithmus zur Verfügung stellen sowie ein Stellsignal abgeben. Alternativ kann ein solcher Rechner eine gegenseitige Parameterbeeinflussung berücksichtende Sollwertvorgabe für einen separaten Regler zur Verfügung stellen.

Ein wesentlicher Prozessparameter für den Biogasanlagenbetrieb ist die Temperatur im Fermenterbehälter, die auch bisher bereits regelmäßig auf einen vorgegebenen Sollwert, durch Veränderung der Heizenergie geregelt wird. Prozessparameter für das vorliegende Biogasanlagen-Regelungsverfahren mit Regel-Biomassen sollen nach Anspruch 5 aber messtechnisch erfassbare Bestandteile und/oder Eigenschaften des zu vergärenden Substrats und/oder entsprechend des erzeugten Biogases sein. Gerade für eine Beeinflussung dieser Prozessparameter eignet sich die erfindungsgemäße Regel-Zudosierung von vorgehaltenen Regel-Biomassen jeweils unterschiedlicher Zusammensetzung.

Nach Anspruch 6 können die Parameter-Istwertgeber Sensoren im Substrat oder im Biogas sein, die an Messumformer angeschlossen sind, die ihrerseits elektrische Istwertsignale liefern. Unter solchen Messumformern werden u. a. auch Analysatorgeräte verstanden, denen automatisiert Gasproben oder Gärsubstratproben für eine kontinuierliche oder quasi kontinuierliche Messung zugeführt werden.

Bei mehreren vorgehaltenen Regel-Biomassen ist es Aufgabe der Regeleinrichtung bzw. eines zugeordneten Rechners festzustellen, welche Regel-Biomasse für die Ausregelung einer Regelabweichung eines bestimmten Prozessparameters geeignet ist. Zudem ist die dafür erforderliche Menge vorzugeben und zuzudosieren.

Für eine solche Mengenzudosierung ist es zweckmäßig nach Anspruch 7 eine oder mehrere Regel-Biomassen in den zugeordneten Regelvorratsbehältern flüssig und/oder granulatartig vorzuhalten und die Regel-Zudosierung mittels an sich bekannter Einrichtungen, wie Pumpen, Pressen oder durch Schnekkentriebe vorzunehmen. Eine durch die Regelung vorgegebene Menge kann je nach Ausführung der Regel-Zudosierung volumetrisch oder über das Gewicht erfasst und zudosiert werden.

Das erfindungsgemäße Regelungsverfahren ist gemäß Anspruch 8 bei mehreren vorgehaltenen Regel-Biomassen mit einer einfachen Vorrichtung durchführbar, wenn mehrere Regelvorratsbehälter in einer Batterie angeordnet sind und die Regelvorratsbehälter über eine gemeinsame Transport-/Förderleitung verbunden sind.

Für einen relativ schnellen Regeleingriff soll nach Anspruch 9 eine Regel-Zudosierung unmittelbar in den Fermenterbehälter erfolgen. Gleichzeitig oder gegebenenfalls für einen langsameren separaten Regeleingriff kann auch die Regelzudosierung in der Basis-Zudosiervorrichtung als Zugabe zur Basis-Biomasse, insbesondere als Zugabe zu einer Feststoff-Biomasse erfolgen.

Die Regelung kann nach Anspruch 10 je nach den Gegebenheiten als Kaskadenregelung aufgebaut werden, bei der die erfindungsgemäße Regelung mittels der Regelzudosierung von Regel-Biomasse als Führungsregelung eingesetzt ist, wobei diese wenigstens einer schnelleren Folgeregelung überlagert ist. Eine solche Folgeregelung kann beispielsweise die an sich bekannte Temperaturregelung für die Fermentertemperatur sein.

Gemäß Anspruch 11 wird vorgeschlagen als Regelalgorithmus einen PID-AIgorithmus zu verwenden, mit einem Proportional-Integral-Differentialanteil. Insbesondere ein Differentialanteil ist hier zweckmäßig, der auf eine erkennbare Regelabweichung schnell mit einer relativ großen Gegensteuerung eingreift, da es sich hier um relativ langsame Regelungsprozesse handelt.

Anhand einer Zeichnung wird die Erfindung näher erläutert.

Es zeigen:
- Fig. 1: eine Biogasanlage mit einer Regelungseinrichtung in einer ersten Ausführungsform, und
- Fig. 2: eine Biogasanlage mit einer Regelungseinrichtung einer zweiten Ausführungsform.

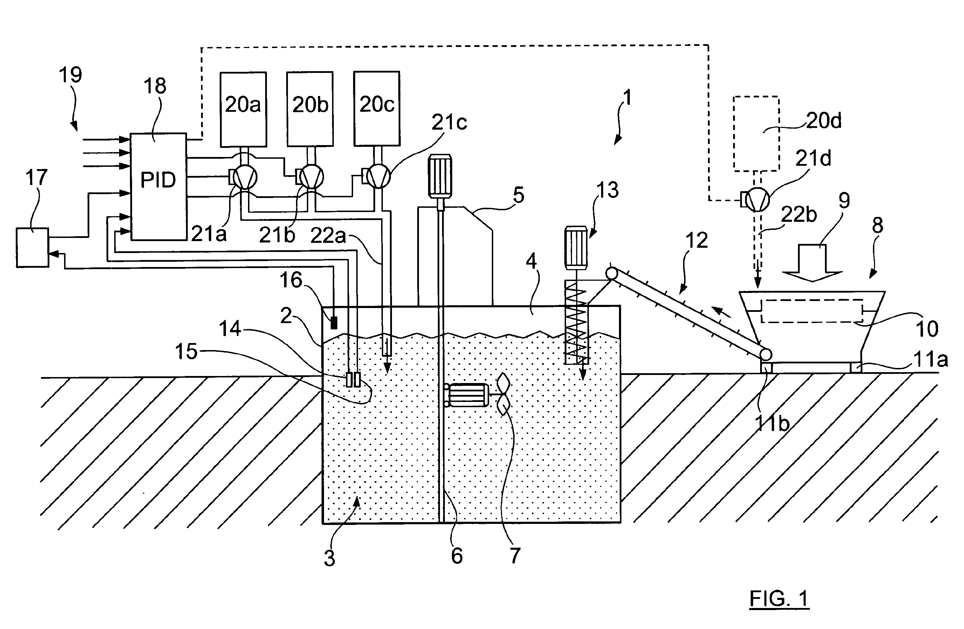

In Fig. 1 ist eine Biogasanlage 1 dargestellt mit einem Fermenterbehälter 2, in dem sich ein zu vergärendes Biomassesubstrat als Basis-Biomasse 3 befindet. Über dem Substratspiegel befindet sich bereits erzeugtes Biogas 4.

Über der Decke des Fermenterbehälters 2 ist ein Serviceschacht 5 angebracht, durch den ein Standrohr 6 in den Fermenterbehälter 2 geführt ist, auf dem höhenverstellbar ein Tauchrührwerk 7 montiert ist.

Eine Basis-Zudosier-Vorrichtung besteht aus einem Misch- und Vorratsbehälter 8, der für eine Zerkleinerung und/oder Mischung zugeführter Biomasse (Pfeil 9) eine Schnecke 10 enthält. Zur Bestimmung des Füllgewichts und der abgegebenen Substratmenge ist eine Wägevorrichtung realisiert, wobei der Misch- und Vorratsbehälter 8 auf Kraftmessdosen 11a, 11b gestellt ist. Die Beschickung erfolgt über ein Förderband 12 in einen Einwurfschacht 13 mit einem Schneckentrieb.

Für die Durchführung des erfindungsgemäßen Regelungsverfahrens sind zur Erfassung von Prozessparametern im Biomassesubstrat 3 beispielhaft zwei Sensoren 14, 15 als Istwertgeber dargestellt. Zudem ist ein weiterer Sensor 16 im Raum für das Biogas 4 dargestellt, über den beispielsweise Gasproben einem Gasanalysator 17 zugeführt werden, dessen Ausgangssignal wiederum als Istwert einem Regler 18 zugeführt wird. Zusätzlich zu den Istwertsignalen aus den Sensoren 14, 15 und dem Gasanalysator 17 sind am Regler 18 zugeordnete Sollwerte 19 vorgebbar. Bei vom Regler ermittelten Regelabweichungen werden über einen PID-Algorithmus Stellsignale für Regeleingriffe in den Gärprozess im Fermenterbehälter 2 ausgegeben.

Dazu sind in einer Batterie drei Regelvorratsbehälter mit jeweils unterschiedlichen Regel-Biomassen 20a, 20b, 20c vorgesehen, die über ausgangsseitige, ansteuerbare Pumpen mit einer Zudosierleitung 22a unmittelbar mit dem Fermenterbehälter 2 verbunden sind. Vom Regler 18 wird für einen Regeleingriff eine der Regel-Biomassen 20a, 20b, 20c ausgewählt. Weiter wird vom Regler 18 eine geeignete Menge vorbestimmt und entsprechend eine vorbestimmte Zeitdauer eine der zugeordneten Pumpen 21 a, 21 b, 21 c für eine Regelzudosierung angesteuert und eingeschaltet.

Strichliert ist eine entsprechende Anordnung für eine Regel-Biomasse 20d mit einer Pumpe 21 d dem Misch- und Vorratsbehälter 8 zugeordnet, in dem je nach den Gegebenheiten über eine Zudosierung 22b die Regel-Biomasse 20d der Basis-Biomasse zugegeben werden kann.

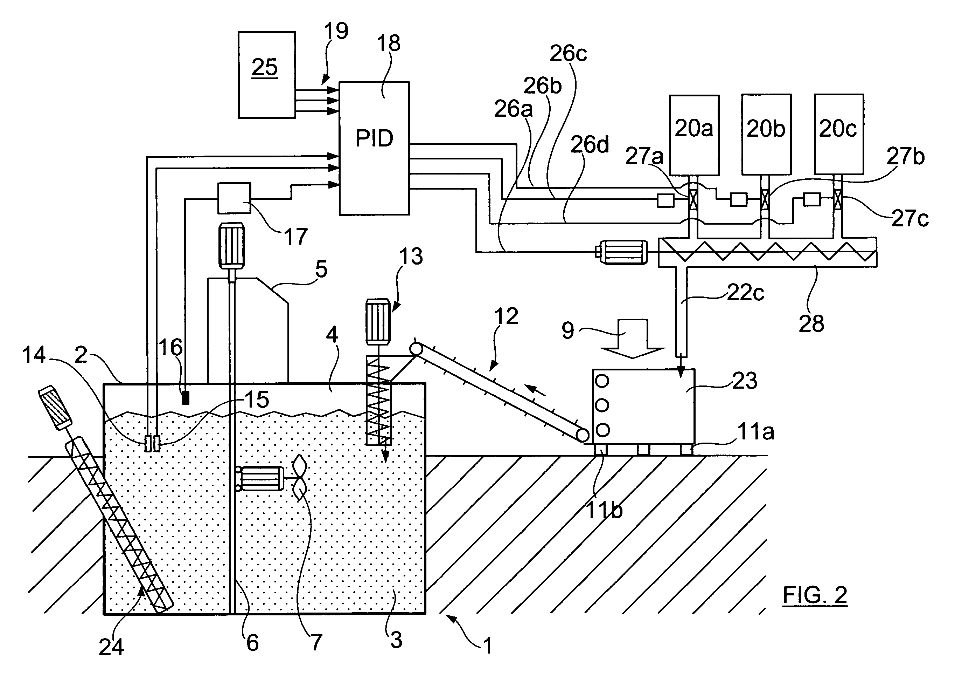

In Fig. 2 ist eine ähnliche Biogasanlage 1 dargestellt. Gleiche Teile sind mit gleichen Bezugszeichen versehen. Die Biogasanlage 1 nach Fig. 2 unterscheidet sich darin, dass anstelle eines Misch- und Vorratsbehälters 8 ein an sich bekannter Zudosierer 23 mit einem Kratzboden und einem Fräswerk am Ausgang für Biofeststoffe verwendet wird. Zudem ist hier eine Austragschnecke 24 dargestellt.

Die Regelung ist hier ähnlich mit Sensoren 14, 15, 16 und einem Gasanalysator 17 als Istwertgeber und einem Regler 18 mit PID-Algorithmus aufgebaut. Die Sollwerte 19 werden hier aber unter Berücksichtigung der gegenseitigen Beeinflussung von Prozessparametern von einem vorgeschalteten Rechner 25 vorgegeben. Eine solche gegenseitige Beeinflussung könnte auch durch eine Rechnereinheit im Regler 18 durch entsprechende Bewertung von Stellsignalen erfolgen, die auf den Ausgangsleitungen 26a, 26b, 26c 26d zur Verfügung gestellt werden.

Auch hier werden in Regel-Vorratsbehältern unterschiedliche Regel-Biomassen 20a, 20b, 20c vorgehalten, die über jeweils ausgangsseitig angeordnete und vom Regler 18 ansteuerbare Ventile 27a, 27b, 27c einer gemeinsamen Förderschnecke 28 mengendosiert zugeführt werden können. Am Förderschneckenende 28 ist eine Zudosierleitung 22c angebracht, mit der die jeweils geeignete Regel-Biomassenmenge dem Zudosierer 23 als Ergänzung zur Basis-Biomasse zugeführt wird.

## Patentansprüche

1. Biogasanlagen-Regelungsverfahren für eine Biogasanlage bestehend aus
einem Fermenterbehälter (2),
- wenigstens einer Basis-Zudosier-Vorrichtung (8, 23) für eine Basis-Biomasse (9), und
- einer Regeleinrichtung zur Regelung wenigstens eines Prozess-Parameters als Regelgröße des Fermentationsprozesses
- mit einem Regler (18), an dem ein zugeordneter Parameter-Sollwert (19) vorgebbar ist, an den zudem ein jeweils zugeordneter Parameter-Istwertgeber (14, 15, 16, 17) angeschlossen ist und der nach einem Soll-IstWertvergleich bei einer festgestellten Regelabweichung ein Stellsignal entsprechend einem vorgebbaren Regelalgorithmus für wenigstens ein nachgeordnetes Stellglied der Regelstrecke abgibt,
**dadurch gekennzeichnet,**
**dass** die Ausregelung einer Regelabweichung bezüglich wenigstens eines Prozessparameters durch eine Regel-Zudosierung einer bestimmten Menge einer zugeordneten und den jeweiligen Prozessparameter beim Fermentationsprozess beeinflussenden Regel-Biomasse (20a, 20b, 20c, 20d) erfolgt, die für einen Regeleingriff in einem Regel-Vorratsbehälter vorgehalten wird und mittels einer Regel-Zudosier-Vorrichtung (21a, 21b, 21c, 21d; 27a, 27b, 27c, 28) als Stellglied entsprechend dem Stellsignal zudosiert wird.

2. Biogasanlagen-Regelungsverfahren nach Anspruch 1, **dadurch gekennzeichnet, dass** zur Regelung eines Prozessparameters wenigstens zwei unterschiedliche Regel-Biomassen (20a, 20b, 20c, 20d) vorgehalten und bedarfsweise geregelt zudosiert werden.

3. Biogasanlagen-Regelungsverfahren nach Anspruch 1 oder 2, **dadurch gekennzeichnet, dass** mehrere Prozessparameter gleichzeitig geregelt werden und die Beeinflussung einer bestimmten vorgegebenen Regel-Biomasse (20a, 20b, 20c, 20d) auf gegebenenfalls mehrere Prozessparameter berücksichtigt wird.

4. Biogasanlagen-Regelungsverfahren nach Anspruch 3, **dadurch gekennzeichnet, dass** die Beeinflussung einer oder mehrerer vorgegebener Regel-Biomassen (20a, 20b, 20c, 20d) auf einen oder mehrere Prozessparameter in einem Rechner (25) als Programm und/oder Kennfeld abgelegt ist und der Rechner selbst die Funktion des Reglers enthält oder für einen separaten Regler (18) geeignete Sollwerte für eine nachgeordnete Regel-Zudosierung vorgibt.

5. Biogasanlagen-Regelungsverfahren nach einem der Ansprüche 1 bis 4, **dadurch gekennzeichnet, dass** Prozessparameter mittels Parameter-Istwertgeber (14, 15, 16, 17) messtechnisch erfassbare Bestandteile und/oder Eigenschaften des zu vergärenden Substrats sind und/oder
dass die Prozessparameter messtechnisch erfassbare Bestandteile und/oder Eigenschaften des erzeugten Biogases sind.

6. Biogasanlagen-Regelungsverfahren nach Anspruch 5, **dadurch gekennzeichnet, dass** Parameter-Istwertgeber aus Sensoren (16) mit nachgeordneten Messumformern (17) gebildet sind, die elektrische Istwertsignale liefern.

7. Biogasanlagen-Regelungsverfahren nach einem der Ansprüche 1 bis 6, **dadurch gekennzeichnet, dass** die eine oder mehrere Regel-Biomassen (20a, 20b, 20c, 20d) in dem oder den zugeordneten Regel-Vorratsbehältern flüssig und/oder granulatartig vorgehalten werden und für die Regelzudosierung mittels Pumpen (21a, 21b, 21c, 21d) und/oder Pressen und/oder durch Schneckentriebe (28) transportiert werden.

8. Biogasanlagen-Regelungsverfahren nach Anspruch 7, **dadurch gekennzeichnet, dass** mehrere Regel-Vorratsbehälter in einer Batterie angeordnet sind mit einer den jeweils behälterzugeordneten Stellgliedern nachgeordneten gemeinsamen Transport-/Förderleitung (22a; 28).

9. Biogasanlagen-Regelungsverfahren nach einem der Ansprüche 1 bis 8, **dadurch gekennzeichnet, dass** die Regel-Zudosierung unmittelbar in den Fermenterbehälter (2) und/oder in die Basis-Zudosier-Vorrichtung (8; 23) als Zugabe zur Basis-Biomasse erfolgt.

10. Biogasanlagen-Regelungsverfahren nach einem der Ansprüche 1 bis 9, **dadurch gekennzeichnet, dass** eine Kaskadenregelung vorgesehen ist, bei der die Regelung mittels der Regel-Zudosierung von Regel-Biomasse (20a, 20b, 20c, 20d) als Führungs-Regelung eingesetzt ist und diese wenigstens einer schnelleren Folgeregelung überlagert ist.

11. Biogasanlagen-Regelungsverfahren nach einem der Ansprüche 1 bis 10,
**dadurch gekennzeichnet, dass** als Regelalgorithmus ein PID-Algorithmus mit einem Proportional-Integral-Differential-Anteil verwendet wird.
